# EUROPÄISCHE PATENTSCHRIFT

(11) **EP 0 937 448 B1**
(45) Veröffentlichungstag und Bekanntmachung des Hinweises auf die Patenterteilung: **09.08.2006**
(21) Anmeldenummer: 99102704.6
(22) Anmeldetag: 17.02.1999
(51) Int. Cl.: A61K 6/00

(54) **Adhäsive Befestigung von dentalen Füllungsmaterialien**
Adhesive attachment of dental filling materials
Fixation adhésive de matières d'obturation dentaire

(30) Priorität: 17.02.1998 DE 19806572
(43) Veröffentlichungstag der Anmeldung: 25.08.1999
(73) Patentinhaber: 3M ESPE AG, 82229 Seefeld (DE)
(72) Erfinder: Frey, Oliver, 82131 Gauting-Königswiesen (DE); Dede, Karsten, 86899 Landsberg (DE); Hansen, Miriam, 81379 München (DE)

(56) Entgegenhaltungen:
- EP-A- 0 301 516
- EP-A- 0 712 622
- EP-A- 1 366 726
- DE-A- 3 414 165
- US-A- 5 525 648
- PATENT ABSTRACTS OF JAPAN vol. 1997, no. 11, 28. November 1997 (1997-11-28) & JP 09 183951 A (TOSOH CORP), 15. Juli 1997 (1997-07-15) -& DATABASE CAPLUS [Online] American Chemical Society; 1997:577102, OSHIMA, NORIAKI: "Acrylic polymer-based UV-curable adhesive for bonding optical disk and metal hub" XP002154123
- HALLER B ET AL: "Dentinpermeabilität nach Behandlung mit Cleanern und Primern" DEUTSCHE ZAHNÄRZTLICHE ZEITSCHRIFT , Bd. 47, Nr. 3, 1992, Seiten 171-175, XP000965523

## Beschreibung

Die vorliegende Erfindung betrifft die Verwendung einer Mischung aus ethylenisch ungesättigten säuregruppenhaltigen Monomeren in einem geeigneten Lösungsmittel in einem Verfahren zur adhäsiven Befestigung von dentalen Füllungsmaterialien in nur einem Schritt.

Zur Befestigung von dentalen Füllungsmaterialien an der Zahnhartsubstanz werden nach heutigem Stand der Technik durch den Behandler folgende Arbeitsschritte durchlaufen:
1.) Anätzen der Zahnhartsubstanz durch eine Säure (Total-etch-Technik),
2.) Auftragen eines sog. Primers, der in die Zahnsubstanz oberflächlich einpenetriert,
3.) Auftragen eines sog. Bondings, welche zusammen mit dem Primer eine Hybridschicht bildet,
4.) Polymerisation des Bondings (z.B. durch Bestrahlen mit Licht oder durch Redox-Reaktion),
5.) Aufbringen des Füllungsmaterials.

Je nach Art und Zusammensetzung dieser Adhäsivsysteme besteht der Primer bzw. das Bonding aus einer oder zwei (vor der Anwendung zu mischender) Flüssigkeiten, die ein- oder zweimal durch den Behandler aufgetragen und zwischenzeitlich, z.B. durch Bestrahlen mit Licht, polymerisiert werden müssen. Darüberhinaus sind diese Adhäsivsysteme in der Regel feuchtigkeitsempfindlich und dürfen nur unter absoluter Trockenlegung (Kofferdamm) verwendet werden. Die gesamte Prozedur zum Befestigen von dentalen Füllmaterialien ist dadurch für den Zahnarzt wesentlich aufwendiger und zeitintensiver, als dies bei Amalgam der Fall war.

Um die Anzahl der anzuwendenden Bestandteile zu verringern, wurden der Primer und das Bonding zu einer Komponente zusammengefasst (sog. Einflaschen-Bondings, z.B. Prime & Bond 2.1® , Fa. Dentsply/Detrey, Dreieich). Dennoch muß zuerst geätzt und anschließend das Einflaschen-Bonding mindestens einmal aufgetragen und anschließend polymerisiert werden, bevor das Füllungsmaterial zur Anwendung kommt. In der Literatur finden sich Hinweise darauf, daß die Effizienz dieser vereinfacht anzuwendenden Adhäsive in Frage zu stellen ist (R. Frankenberger, N. Krämer, J. Sindel; Dtsch Zahnärztl Z 51 (1996), 556-560).

Eine andere Vereinfachung des oben beschriebenen Verfahrens zur adhäsiven Befestigung von Füllungsmaterialien besteht darin, den Primer und das Ätzmittel zu einer Komponente zu kombinieren (sog. selbstätzende Primer; z.B. Etch & Prime 3.0® , Fa. Degussa; Clearfil-Linerbond 2.0® , Fa. Kuraray, Osaka). Diese müssen zwar nur noch aufgetragen und nicht mehr abgespült werden, ihnen folgt aber entweder noch das zu polymerisierende Bonding des eingangs beschriebenen Verfahrens (z.B. Clearfil-Linerbond 2.0® , Fa. Kuraray, Osaka) oder zumindest aber ein Vernetzungsschritt zur Polymerisation des selbstätzenden Primers, bevor das Füllungsmaterial verwendet werden kann.

In der DE-A-34 14 163 sind selbstätzende Mischungen von Phosphorsäurehemaestern mit einem Photoinitiator in Aceton als Lösungsmittel offenbart. Um einen Haftverbund zur Zahnhartsubstanz herstellen zu können, lehrt aber auch diese Schrift, vor dem eigentlichen Füllmaterial einen weiteren Haftvermittler aufzutragen, der zunächst polymerisiert werden muß.

Selbstätzende Adhäsivmischungen, die vor dem Auftragen des Füllungsmaterials nicht mehr polymerisiert werden müssen sind in EP-A-0 183 027 beschrieben. Sie benötigen aber einen zusätzlichen Katalysator in Form einer Sulfinsäure und/oder deren Salz, um eine ausreichende Haftung am Dentin zu erreichen. Belege dafür, daß eine gute Schmelzhaftung ohne vorherigen Ätzschritt erreicht wird, sind der Schrift nicht zu entnehmen.

Ebenfalls selbstätzende Gemische sind in der EP-A-0 088 527 offenbart, die als saure Komponente jedoch relativ aufwendig herzustellende und damit vergleichsweise teure Phosphorsäureester enthalten müssen. Diese Verbindungen tragen dabei mindestens drei ethylenisch ungesättigte Gruppen und enthalten keine weitere Initiatorkomponente. Die angegebenen Haftwerte für Dentin und Schmelz sind gering im Vergleich zu denjenigen, welche durch Vorbehandlung der Zahnhartsubstanz durch Ätzen und Bonding erzielbar sind.

In der US-A-5 264 513 sind Mischungen beschrieben, die neben Wasser, einer ungesättigten polymerisierbaren Säure und einem Katalysator noch ungesättigte polymerisierbare hydroxylgruppenhaltige Monomere enthalten müssen, um eine ausreichende Dentin- und Schmelzhaftung zu gewährleisten. Diese hydroxylgruppenhaltigen Monomere, insbesondere das bevorzugte 2-HEMA, besitzen ein hohes Sensibilisierungspotential und stellen somit für den Anwender dieser Mischungen eine Gefährdung dar.

In der US-A-5 304 585 sind Adhäsive auf der Basis von polymerisierbaren Phosphorsäurehalogeniden beschrieben, die auch ohne vorheriges Ätzen zu ausreichendem Haftverbund am Dentin führen. Um jedoch optimale Haftkräfte am Schmelz zu erzielen, ist ein vorheriger seperater Ätzschritt erforderlich.

US 5 525 648 offenbart ein mehrstufiges Verfahren, in dem ein Primer auf die Zahnhartsubstanz aufgebracht und getrocknet wird und der erhaltene Film in einer Schicht aus zusätzlichem Filmbildner überschichtet wird.

EP 0 301 516 A offenbart photopolymerisierbare dentale Adhäsivzusammensetzungen, die erst durch Bestrahlung ihre inhärenten Adhäsionseigenschaften ausbilden und folglich erst dann ein Füllungsmaterial überschichtet werden kann.

EP 0 712 622 A beschreibt ein Verfahren, bestehend aus dem Auftragen eines Vorbehandlungsmittels auf die Zahnoberfläche, dessen Trocknung, nachfolgender Aufbringung eines Adhäsivs, Härtung dieses Adhäsivs und schließlich Aufbringen eines dentalen Füllungsmaterials und dessen Härtung.

DE 34 14 165 A beschreibt das Aufbringen eines Haftmittels in dünner Schicht auf ungeätztes Dentin und geätzten Schmelz und dessen Aushärtung mit UV-Licht.

Daneben sind in der Literatur eine Reihe weiterer Adhäsivmischungen beschrieben, die zwar selbstätzend wirken, jedoch vor der Anwendung des Füllungsmaterials entweder mehrmals aufgetragen und/oder polymerisiert werden müssen.

Aufgabe der vorliegenden Erfindung ist es, Adhäsivmischungen zur Befestigung von dentalen Füllungsmaterialien zur Verfügung zu stellen, welche in einem Schritt aufgetragen und ohne weitere Nachbehandlung mit polymerisierbarem Füllungsmaterial überschichtet werden und dabei gleichzeitig zu guten Haftwerten am Dentin und sehr guten Haftwerten am Schmelz führen. Diese Adhäsivmischungen sollten dabei aus möglichst wenigen Komponenten bestehen, preisgünstig zugänglich und für den Anwender gefahrlos zu verwenden sein.

Gelöst wird die Aufgabe durch die Verwendung einer Mischung, die
i) 10 bis 90 Gewichtsteile mindestens eines einfach oder mehrfach ethylenisch ungesättigtem Phosphorsäureesters,
ii) 5 bis 85 Gewichtsteile eines Lösungsmittels,
iii) 0,01 bis 5 Gewichtsteile eines Initiators, der freie Radikale bilden kann, und
iv) 0 bis 10 Gewichtsteile übliche Hilfs- und Zusatzstoffe
umfaßt, wobei unmittelbar nach dem Auftragen der Mischung auf die unbehandelte Zahnhartsubstanz ohne einen zwischenzeitlichen Polymerisationsschritt mit polymerisierbarem Füllungsmaterial überschichtet wird.

Überraschenderweise wurde gefunden, daß derartige Mischungen bereits durch bloßes einmaliges Auftragen auf unbehandeltes Dentin bzw. unbehandelten Schmelz und ohne zwischenzeitlichen Polymerisationsschritt zu gleich guten Haftverbunden mit dentalen Füllungsmaterialien führen, wie mit Ätzmittel, Primer und Bonding vorbehandelte Zahnhartsubstanz. Der Vorteil für den Anwender ist eine Einsparung von mehreren Arbeitsschritten und damit verbunden ein Zeitgewinn, welcher die Behandlung auch für den Patienten weniger unangenehm gestaltet. Trotz des deutlich verkürzten Arbeitsganges zeigen die hervorragenden Haftwerte, insbesondere am Zahnschmelz, daß die Qualität der Restaurationen nicht schlechter als bei herkömmlichen Befestigungsverfahren ist.

Im folgenden wird die Erfindung näher beschrieben:

Die adhäsive Mischung, welche die beschriebenen Vorteile bei erfindungsgemäßer Verwendung aufweist, enthält als Bestandteil i) 10 bis 90 Gewichtsteile, vorzugsweise 30 bis 85 Gewichtsteile, mindestens eines einfach oder mehrfach ungesättigten Phosphorsäureesters. Geeignete phosphorsäuregruppenhaltige Monomere sind bekannt und z.B. in den US-A-4 182 035, US-A-4 222 780, US-A-4 235 633, US-A-4 359 117 und US-A-4 368 043 sowie in der EP-A-0 084 407 beschrieben. Bevorzugt verwendet werden in der Adhäsivmischung ethylenisch ungesättigte Phosphorsäureester gemäß folgender Formel: 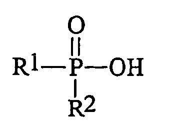 in welcher R¹ und R² unabhängig voneinander für OH oder für 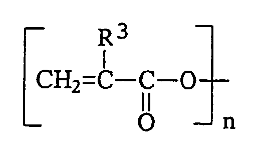 stehen, wobei R³ Wasserstoff oder C₁- bis C₃-Alkyl bedeutet und n eine ganze Zahl ≥ 1 ist,
mit der Maßgabe daß mindestens einer der Reste R¹ oder R² ungleich OH ist.

Bestandteil ii) der adhäsiven Mischung ist ein Lösungsmittel, welches in der Lage ist, die Bestandteile i) und iii) vollständig zu lösen. Geeignet sind solche Lösungsmittel, welche ausreichend flüchtig und ohne Gefährdung für den Anwender anzuwenden sind. Beispiele hierfür sind leichtflüchtige Alkohole, wie Ethanol, Propanol, oder Isopropanol, sowie flüssige, wenig giftige Ketone, wie z.B. Aceton. Besonders bevorzugt als Lösungsmittel ist Wasser. Bestandteil ii) ist in den adhäsiven Gemischen zu 5 bis 85 Gewichtsteilen, bevorzugt zu 10 bis 80 Gewichtsteilen, enthalten.

Radiakalbildende Katalysatoren gemäß Bestandteil iii), welche in den Mischungen enthalten sein müssen, können durch UV- oder sichtbares Licht aktivierbare Substanzen sein, wie z.B. Benzoinalkylether, Benzilketale, Acylphosphinoxide oder aliphatische und aromatische 1,2-Diketonverbindungen, z.B. Campherchinon, wobei die Katalysatoraktivität durch Zusatz von Aktivatoren, wie tertiären Aminen oder organischen Phosphiten, in an sich bekannter Weise beschleunigt werden kann.

Geeignete Initiatorsysteme zur Auslösung der radikalischen Polymerisation über einen Redox-Mechanismus sind beispielsweise die Systeme Peroxid/Amin oder Peroxid/Barbitursäurederivate u. dergl. Bei Verwendung solcher Initiatorsysteme ist es zweckmäßig, einen Initiator (z.B. Peroxid) und eine Katalysatorkomponente (z.B. Amin) getrennt bereitzuhalten. Die beiden Komponenten werden dann kurz vor ihrer Anwendung miteinander homogen vermischt. Das Füllungsmaterial wird aber auch in diesem Fall unmittelbar nach dem Auftragen der Adhäsivmischung aufgebracht, also noch bevor eine Polymerisation der Adhäsivmischung vollständig abgelaufen ist. Zudem besteht die Möglichkeit, daß in der Adhäsivmischung nur eine Komponente des Redoxinitiators (z.B. Peroxid) und die andere Komponente (z.B. Amin) im Füllungsmaterial enthalten ist, wodurch die Polymerisation erst nach Aufbringen der Füllungsmasse gestartet wird.

Die Katalysatorkomponente iii) ist in den Mischungen zu 0,01 bis 5 Gewichtsteilen, bevorzugt zu 0,1 bis 2,5 Gewichtsteilen, enthalten.

Geeignete Hilfsstoffe gemäß Komponente iv) können beispielsweise üblicherweise auf dem Dentalgebiet eingesetzte Stabilisatoren, Pigmente oder Füllstoffe sein. Geeignete Füllstoffe sind z.B. feingemahlene Quarzpulver, Gläser, sowie fluoridhaltige Stoffe, wie Yttriumtrifluorid oder Natriumfluorid. Geeignete Stabilisatoren können z.B. Tenside sein.

Die offenbarten adhäsiven Mischungen können einkomponentig oder für den Fall, daß einzelne Inhaltsstoffe über einen längeren Lagerzeitraum nicht miteinander kompatibel sind, auch räumlich getrennt voneinander aufbewahrt werden. Die Erfindung lässt sich auch dann ausführen, wenn die beschriebenen Komponenten erst unmittelbar vor der Anwendung miteinander vermischt werden.

Im folgenden soll die Erfindung an Hand von Beispielen näher erläutert werden:

### Referenzbeispiel 1:

### Haftmessung an Rinderzähnen durch adhäsive Befestigung eines Füllungsmaterials durch All-etch"-Technik:

Die Überprüfung des Haftverbundes erfolgte durch einen Haftabzugsversuch an Rinderzähnen. Pro Versuch wurden 5 frisch extrahierte Rinderzähne mittels Schleifpapier soweit abgeschliffen, daß eine ausreichend große freiliegende Schmelz- bzw. Dentinfläche entstand. Auf diese Flächen wurden jeweils Wachsplättchen mit einem ausgestanzten Loch von 4 mm geklebt, um eine standardisierte Haftfläche zu erhalten. Die so erhaltenen Prüfkörper wurden in Anlehnung an das praxisübliche Vorgehen ("all-etch Technik") mittels einer üblichen Phosphorsäurelösung (Ätzgel Minitip® , Fa. ESPE Dental-Medizin, Seefeld) für 15 Sekunden angeätzt und anschließend mit Wasser abgespült. Auf die derart vorbereiteten Schmelz- bzw. Dentinflächen wurde der Haftvermittler (Hytac OSB® , Fa. ESPE) aufgetragen und für 30 Sekunden mittels eines Microbrushes einmassiert. Anschließend wurde kurz mit Druckluft verblasen und mittels eines Lichtpolymerisationsgerätes (Elipar® , Fa. ESPE) für 10 Sekunden polymerisiert. Dann wurde nochmals der gleiche Haftvermittler aufgetragen, sofort verblasen und erneut für 10 Sekunden lichtgehärtet. Anschließend wurde ein dentales Füllungsmaterial (Hytac® , Fa. ESPE) in zwei Schichten in die Aussparungen der Wachsplättchen eingebracht und jede Schicht gemäß den Herstellerangaben auspolymerisiert. Nach 24-stündiger Lagerung bei 36°C und 100% Luftfeuchtigkeit wurden die Wachsplättchen entfernt und die Prüfkörper in einem Zugversuch (Zwick Universalprüfmaschine) abgezogen. Die dabei ermittelten Schmelz- bzw. Dentinhaftwerte sind Tabelle 1 zu entnehmen.

### Herstellungsbeispiel 1:

### Herstellung einer erfindungsgemäßen Adhäsivmischung:

70,0g Aceton, 20,0g einer Mischung aus Phosphorsäuredihemaester und Phosphorsäuremonohemaester (Mischungsverhältnis 1:1,5; herstellbar durch Vermischen von 11,4g 2-Hydroxyethylmethacrylat mit 8,75g Phosphorpentaoxid), 10,0g entionisiertes Wasser und 0,2g Bis-(2,6-dichlorbenzoyl)-(4-butylphenyl)-phosphinoxid wurden solange gerührt, bis ein klare, leicht gelbliche Lösung entstanden war.

### Herstellungseispiel 2:

### Herstellung einer erfindungsgemäßen Adhäsivmischung:

80,0 g einer Mischung aus Phosphorsäuredihemaester und Phosphorsäuremonohemaester (Mischungsverhältnis 1:1,5; Herstellbar durch Vermischen von 11,4 g 2-Hydroxyethylmethacrylat mit 8,75 g Phosphorpentaoxid), 20g entionisiertes Wasser und 0,2g Bis-(2,6-dichlorbenzoyl)-(4-butylphenyl)-phosphinoxid wurden solange gerührt, bis ein klare, leicht gelbliche Lösung entstanden war.

### Beispiel 1:

### Haftmessung an Rinderzähnen durch adhäsive Befestigung eines Füllungsmaterials mit einer erfindungsgemäßen Adhäsivmischung:

Die Vorbereitung der Rinderzähne zu Ermittlung der Schmelz- bzw. Dentinhaftung erfolgte in der gleichen Weise, wie in Beispiel 1 beschrieben. Auf die standardisierte Haftfläche wurde ohne vorheriges Ätzen die in den Herstellungsbeispielen 1 und 2 beschriebenen Lösungen mit einem Microbrush aufgebracht und für 15 Sekunden einmassiert. Anschließend wurde kurz mit Druckluft verblasen und daraufhin gleich die erste Schicht des dentalen Füllungsmaterials (Hytac® , Fa. ESPE) aufgebracht und lichtgehärtet. Nach Auftragen der zweiten Schicht Füllungsmaterial und Lichthärtung wurden die Prüfkörper 24 Stunden bei 36°C und 100% Luftfeuchtigkeit gelagert. Anschließend wurden die Wachsplättchen entfernt und die Prüfkörper in einem Zugversuch (Zwick Universalprüfmaschine) abgezogen. Die dabei ermittelten Schmelz- bzw. Dentinhaftwerte sind Tabelle 1 zu entnehmen.

### Referenzbeispiel 2:

### Haftmessung an Rinderzähnen durch adhäsive Befestigung eines Füllungsmaterials ohne vorheriges

### Ätzen mit herkömmlichem Haftvermittler.

Die Vorbereitung der Rinderzähne zu Ermittlung der Schmelz- bzw. Dentinhaftung erfolgte in der gleichen Weise, wie in Beispiel 1 beschrieben. Auf die standardisierte Haftfläche wurde ohne vorheriges Ätzen der Haftvermittler (Hytac OSB® , Fa. ESPE) aufgetragen und für 30 Sekunden mittels eines Microbrushes einmassiert. Anschließend wurde kurz mit Druckluft verblasen und mittels eines Lichtpolymerisationsgerätes (Elipar® , Fa. ESPE) für 10 Sekunden polymerisiert. Dann wurde nochmals der gleiche Haftvermittler aufgetragen, sofort verblasen und erneut für 10 Sekunden lichtgehärtet. Anschließend wurde ein dentales Füllungsmaterial (Hytac® , Fa. ESPE) in zwei Schichten in die Aussparungen der Wachsplättchen eingebracht und jede Schicht gemäß den Herstellerangaben auspolymerisiert. Nach 24-stündiger Lagerung bei 36°C und 100% Luftfeuchtigkeit wurden die Wachsplättchen entfernt und die Prüfkörper in einem Zugversuch (Zwick Universalprüfmaschine) abgezogen. Die dabei ermittelten Schmelz- bzw. Dentinhaftwerte (Mittelwerte aus je 5 Messungen) sind Tabelle 1 zu entnehmen.

**Tabelle 1: Schmelz- bzw. Dentinhaftung der in den Beispielen beschriebenen Adhäsivmischungen:**

| Adhäsivmischung aus Beispiel-Nr. | Schmelzhaftung [MPa]* | Dentinhaftung [MPa]* |
|---|---|---|
| Referenzbeispiel 1 | 11,7 | 4,8 |

| Beispiel 1 | | |
|---|---|---|
| Mischung des Herstellungsbeispiels 1 | 15,2 | 5,3 |
| Mischung des Herstellungsbeispiels 2 | 15,5 | 6,2 |
| Referenzbeispiel 2 | 3,7 | 5,8 |

| | | |
|---|---|---|
| *Mittelwert aus je 5 Messungen | | |

Wie Tabelle 1 zu entnehmen ist, zeigen die erfindungsgemäß eingesetzten Mischungen gleich gute Dentinhaftwerte wie die Adhäsive der Referenzbeispiele. Die Schmelzhaftung mit den erfindungsgemäß eingesetzten Mischungen ist deutlich höher als die von Referenzbeispiel 2, obwohl hier zwischenzeitlich polymerisiert wurde und auch noch besser als die von Referenzbeispiel 1, bei welchem die Schmelzoberfläche zunächst angeätzt werden musste, um diese Werte zu erreichen.

Es sei an dieser Stelle nochmals darauf hingewiesen, daß die guten Dentinhaftwerte und die ausgezeichneten Schmelzhaftwerte bei gleichzeitig deutlich vereinfachter Anwendung für den Behandler und deutlich angenehmeren Behandlungsbedingungen für den Patienten erzielt werden.

## Patentansprüche

1. Verwendung einer Mischung, umfassend
i) 10 bis 90 Gewichtsteile mindestens eines einfach oder mehrfach ethylenisch ungesättigtem Phosphorsäureesters,
ii) 5 bis 85 Gewichtsteile eines Lösungsmittels,
iii) 0,01 bis 5 Gewichtsteile eines Initiators, der freie Radikale bilden kann, und
iv) 0 bis 10 Gewichtsteile übliche Hilfs- und Zusatzstoffe
zur Herstellung eines Mittels zur adhäsiven Befestigung von dentalen Füllungsmaterialien, bei welcher unmittelbar nach dem Aufbringen der Mischung auf die unbehandelte Zahnhartsubstanz ohne einen zwischen zeitlichen Polymerisationsschritt mit polymerisierbarem Füllungsmaterial überschichtet wird.

2. Verwendung nach Anspruch 1, **dadurch gekennzeichnet, daß** Bestandteil i) ein ethylenisch ungesättigter Phosphorsäureester der folgenden Formel ist: 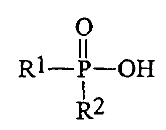 in welcher R¹ und R² unabhängig voneinander für OH oder für 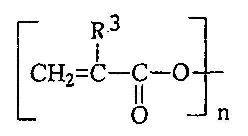 stehen, wobei R³ Wasserstoff oder C₁- bis C₃-Alkyl bedeutet und n eine ganze Zahl ≥ 1 ist, mit der Maßgabe, daß mindestens einer der Reste R¹ oder R² ungleich OH ist.

3. Verwendung nach den Ansprüchen 1 und 2, **dadurch gekennzeichnet, daß** Bestandteil ii) Wasser oder Aceton ist.

4. Verwendung nach den Ansprüchen 1 bis 3, **dadurch gekennzeichnet, daß** Bestandteil iii) ein Photoinitiator ist.

## Claims

1. Use of a mixture comprising
i) 10 to 90 parts by weight of at least one singly or multiply ethylenically unsaturated phosphoric acid ester,
ii) 5 to 85 parts by weight of a solvent,
iii) 0.01 to 5 parts by weight of an initiator which can form free radicals, and
iv) 0 to 10 parts by weight of customary auxiliaries and additives, for the production of a composition for the adhesive securing of dental filling material wherein immediately after the application of the mixture to the untreated hard tooth substance it is coated with polymerizable filling material without an intermediate polymerization step.

2. Use according to Claim 1, **characterized in that** constituent i) is an ethylenically unsaturated phosphoric acid ester of the following formula: 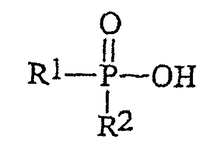 in which R¹ and R² stand independently of each other for OH or for 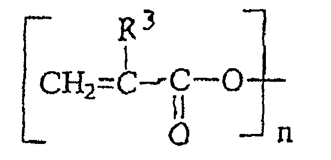 where R³ represents hydrogen or C₁- to C₃-alkyl and n is a whole number ≥ 1, on condition that at least one of the radicals R¹ or R² does not equal OH.

3. Use according to Claim 1 or 2, **characterized in that** constituent ii) is water or acetone.

4. Use according to any of Claims 1 to 3, **characterized in that** constituent iii) is a photoinitiator.

## Revendications

1. Utilisation d'un mélange comprenant
i) 10 à 90 parties en poids d'au moins un ester d'acide phosphorique une ou plusieurs fois éthyléniquement insaturé,
ii) 5 à 85 parties en poids d'un solvant,
iii) 0,01 à 5 parties en poids d'un amorceur, qui peut former des radicaux libres, et
iv) 0 à 10 parties en poids d'additifs et adjuvants usuels,
pour la préparation d'un agent pour la fixation adhésive de matières d'obturation dentaire, dans lequel on recouvre avec une matière d'obturation dentaire polymérisable sans étape de polymérisation intermédiaire immédiatement après le dépôt du mélange sur la substance dure de la dent non traitée.

2. Utilisation selon la revendication 1, **caractérisée en ce que** le constituant i) est un ester d'acide phosphorique éthyléniquement insaturé de formule : 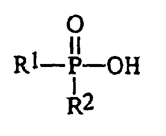 dans laquelle R¹ et R² représentent indépendamment l'un de l'autre OH ou 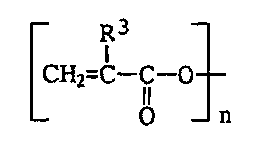 R³ représentant l'hydrogène ou un alkyle en C₁ à C₃ et n étant un nombre entier ≥ 1,
sous réserve qu'au moins l'un des radicaux R¹ ou R² soit différent de OH.

3. Utilisation selon les revendications 1 et 2, **caractérisée en ce que** le constituant ii) est l'eau ou l'acétone.

4. Utilisation selon les revendications 1 à 3, **caractérisée en ce que** le constituant iii) est un photo-amorceur.
